# EUROPÄISCHE PATENTSCHRIFT

(11) **EP 0 629 666 B1**
(45) Veröffentlichungstag und Bekanntmachung des Hinweises auf die Patenterteilung: **02.12.1998**
(21) Anmeldenummer: 94109016.9
(22) Anmeldetag: 13.06.1994
(51) Int. Cl.: C09B 62/085, C09B 62/51

(54) **Wasserlösliche Azoverbindungen, Verfahren zu ihrer Herstellung und ihre Verwendung als Farbstoffe**
Water-soluble azo compounds, process for their preparation and their use as dyestuffs
Composés azoiques solubles dans l'eau, procédé pour leur preparation et leur utilisation comme colorants

(30) Priorität: 17.06.1993 DE 4320151
(43) Veröffentlichungstag der Anmeldung: 21.12.1994
(73) Patentinhaber: DyStar Textilfarben GmbH & Co. Deutschland KG, 60318 Frankfurt am Main (DE)
(72) Erfinder: Dannheim, Jörg, Dr., D-60489 Frankfurt/Main (DE); Oehme, Dieter, D-65439 Flörsheim (DE); Tappe, Horst, Dr., D-63128 Dietzenbach (DE)

(56) Entgegenhaltungen:
- EP-A- 0 177 445
- EP-A- 0 308 787

## Beschreibung

Aus den U.S.-Patentschriften Nrs. 4 649 193 und 5 097 021 sind Monoazofarbstoffe bekannt, die in der Kupplungskomponente einen aminosubstituierten Fluortriazinrest und in der Diazokomponente einen faserreaktiven Rest der Vinylsulfonreihe enthalten. Sie besitzen zwar gute faserreaktive und anwendungstechnische Eigenschaften, jedoch fordert die Praxis des Färbens mit faserreaktiven Farbstoffen weiterhin Verbesserungen in der Qualität der Eigenschaften solcher Farbstoffe und der mit ihnen erhältlichen Färbungen und Drucke und ebenso eine verbesserte Wirtschaftlichkeit des Färbeprozesses bei deren Anwendung. Es bestand infolgedessen weiterhin ein Bedarf nach neuen faserreaktiven Farbstoffen, mir denen eine Verbesserung des Standes der Technik erzielt werden kann.

Mit der vorliegenden Erfindung wurden nunmehr neue Monoazoverbindungen mit faserreaktiven Eigenschaften gefunden, die der allgemeinen Formel (1) 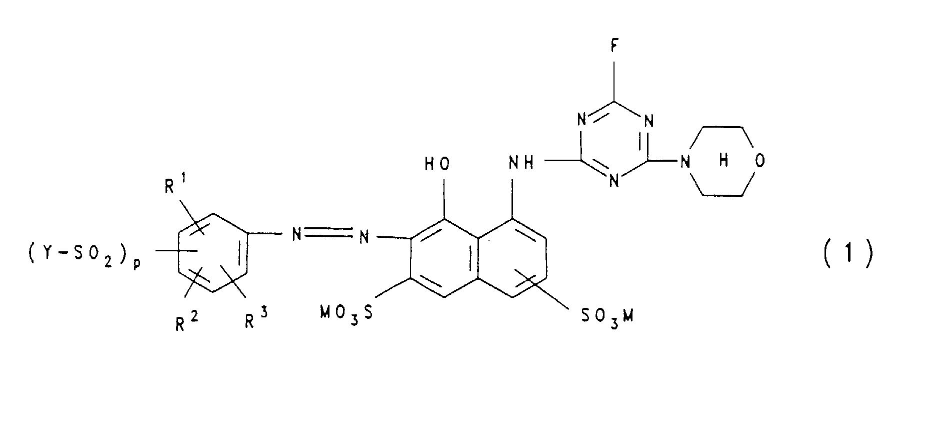 entsprechen. In dieser Formel bedeuten:
- M: ist Wasserstoff oder ein Alkalimetall, wie Natrium, Kalium oder Lithium;
- R¹: ist Wasserstoff oder Sulfo;
- R²: ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Sulfo, Hydroxy, Halogen, wie Chlor, Brom und Fluor, oder Carboxy, bevorzugt Wasserstoff, Methyl, Methoxy oder Ethoxy;
- R³: ist Wasserstoff, Halogen, wie Chlor und Brom, Alkyl von 1 bis 4 C-Atomen, wie Ethyl und insbesondere Methyl, oder Alkoxy von 1 bis 4 C-Atomen, wie Ethoxy und insbesondere Methoxy, bevorzugt Wasserstoff, Methoxy oder Ethoxy;
- Y: ist Vinyl oder ist Ethyl, das in β-Stellung einen Substituenten enthält, der durch Alkali unter Bildung der Vinylgruppe eliminierbar ist;
- p: ist die Zahl 1 oder 2;
die eine Gruppe -SO₃M steht in para- oder meta-Stellung zur Aminogruppe an den Naphthalinring gebunden.

Die Monoazoverbindungen der allgemeinen Formel (1) können in Form der freien Säure als auch in Form ihrer Salze vorliegen. Bevorzugt sind sie in Form ihrer Salze und finden bevorzugt in Form der Salze Verwendung zum Färben und Bedrucken von hydroxy- und/oder carbonamidgruppenhaltigen Materialien, insbesondere Fasermaterialien.

In der allgemeinen Formel (1) und ebenso in den nachfolgenden allgemeinen Formeln können die einzelnen Formelglieder im Rahmen ihrer Bedeutung zueinander gleiche oder voneinander verschiedene Bedeutungen haben.

Alkalisch eliminierbare Substituenten in der Ethylgruppe des Restes Y sind beispielsweise Sulfato, Thiosulfato, Phosphato, Alkanoyloxy von 2 bis 5 C-Atomen, wie Acetyloxy, Benzyloxy, Sulfobenzyloxy, p-Toluolsulfonyloxy, Halogen, wie Chlor, und Dialkylamino mit Alkylresten von jeweils 1 bis 4 C-Atomen, wie Dimethylamino und Diethylamino, bevorzugt hiervon Thiosulfato, Acetyloxy und insbesondere Sulfato. Bevorzugt ist Y ebenso Vinyl.

Die Gruppen "Sulfo", "Carboxy", "Thiosulfato", "Phosphato" und "Sulfato" schließen sowohl deren Säureform als auch deren Salzform ein. Demgemäß bedeuten Sulfogruppen Gruppen entsprechend der allgemeinen Formel -SO₃M, Thiosulfatogruppen Gruppen entsprechend der allgemeinen Formel -S-SO₃M, Carboxygruppen Gruppen entsprechend der allgemeinen Formel -COOM, Phosphatogruppen Gruppen entsprechend der allgemeinen Formel -OPO₃M₂ und Sulfatogruppen Gruppen entsprechend der allgemeinen Formel -OSO₃M, in welchen M die oben genannte Bedeutung besitzt.

Diazokomponenten entsprechend den Resten der allgemeinen Formel (2)

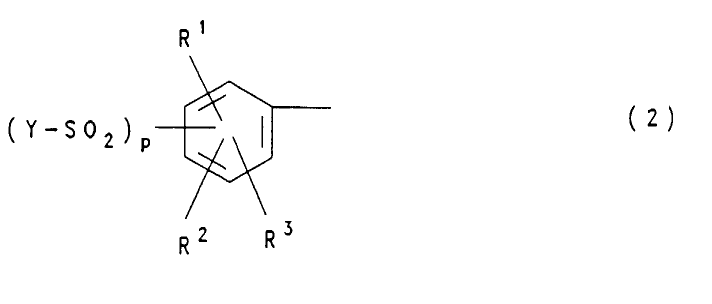

in den Monoazoverbindungen der allgemeinen Formel (1) sind beispielsweise 4-(β-Sulfatoethylsulfonyl)-phenyl, 4-(β-Thiosulfatoethylsulfonyl)-phenyl, 4-Vinylsulfonyl-phenyl, 4-(β-Chlorethylsulfonyl)-phenyl, 3-(β-Sulfatoethylsulfonyl)-phenyl, 3-Vinylsulfonyl-phenyl, 2-Methoxy-5-(β-sulfatoethylsulfonyl)-phenyl, 2-Methoxy-5-(β-thiosulfatoethylsulfonyl)-phenyl, 2-Methoxy-5-vinylsulfonyl-phenyl, 4-Methoxy-3-(β-sulfatoethylsulfonyl)-phenyl, 4-Methoxy-3-vinylsulfonyl-phenyl, 2,5-Dimethoxy-4-(β-sulfatoethylsulfonyl)-phenyl, 2,5-Dimethoxy-4-vinylsulfonyl-phenyl, 2,5-Dimethoxy-4-(β-sulfatoethylsulfonyl)-phenyl, 2-Carboxy-5-(β-sulfatoethylsulfonyl)-phenyl, 2-Carboxy-5-vinylsulfonylphenyl, 2-Sulfo-4-(β-sulfatoethylsulfonyl)-phenyl, 2-Sulfo-4-vinylsulfonyl-phenyl, 2,4-Disulfo-5-vinylsulfonyl-phenyl, 2-Hydroxy-5-β-sulfatoethylsulfonyl-phenyl, 2-Hydroxy-4-(β-sulfatoethylsulfonyl)-phenyl, 3-Sulfo-2-hydroxy-5-(β-sulfatoethylsulfonyl)-phenyl, 2-Brom-4-(β-sulfatoethylsulfonyl)-phenyl und 2,6-Dichlor-4-(β-sulfatoethylsulfonyl)-phenyl.

Die vorliegende Erfindung betrifft weiterhin ein Verfahren zur Herstellung der erfindungsgemäßen Monoazoverbindungen der allgemeinen Formel (1), das dadurch gekennzeichnet ist, daß man eine Verbindung der allgemeinen Formel (3) 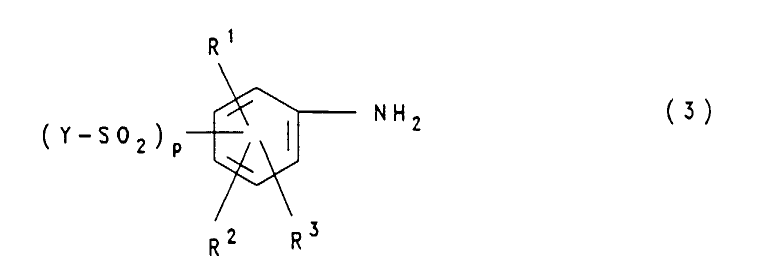 in welcher Y, R¹, R², R³ und p die oben genannten Bedeutungen haben, diazotiert und mit einer Verbindung entsprechend der allgemeinen Formel (4) 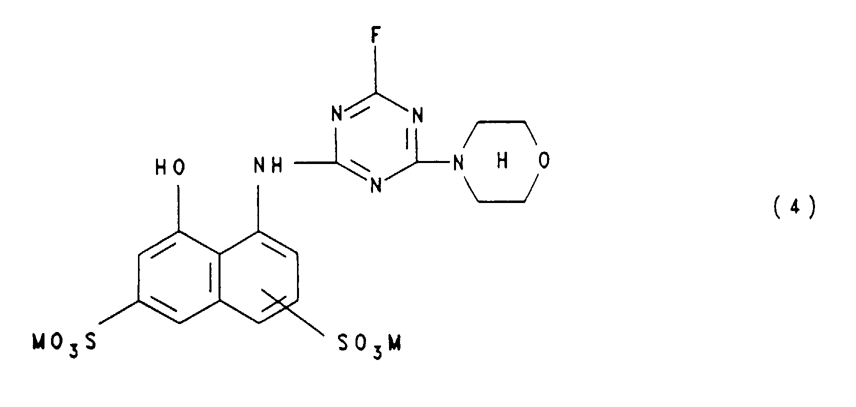 in welcher M die oben genannte Bedeutung besitzt, kuppelt.

Die Diazotierungsreaktion der Verbindung der allgemeinen Formel (3) erfolgt in an und für sich bekannter Verfahrensweise, so in wäßrigem Medium bei einer Temperatur zwischen -5°C und + 15°C und einem pH-Wert unterhalb von 2 mittels einer starken Säure, wie Schwefelsäure oder Salzsäure, und einem Alkalinitrit. Die Kupplungsreaktion erfolgt in wäßriger Lösung oder in einem wäßrigen-organischen Medium, wobei der organische Lösemittelanteil mit Wasser mischbar und gegenüber den Reaktanten inert ist, wie beispielsweise Aceton, Dimethylformamid, Dimethylsulfoxid und N-Methyl-pyrrolidon. Die Kupplungsreaktion erfolgt in der Regel bei einer Temperatur zwischen 0 und 20°C, bevorzugt zwischen 5 und 15°C und bei einem pH-Wert zwischen 2 und 7, bevorzgt zwischen 4 und 6.

Die faserreaktiven Anilin-Ausgangsverbindungen der allgemeinen Formel (3) sind allgemein bekannt und zahlreich in der Literatur beschrieben. Die als Kupplungskomponente dienende Ausgangsverbindung der allgemeinen Formel (4) und deren Herstellung ist aus der anfangs erwähnten U.S. Patentschrift Nr. 5 097 021 bekannt.

Die Abscheidung der erfindungsgemäß hergestellten Verbindungen der allgemeinen Formel (1) - im nachfolgenden Verbindungen (1) genannt - aus den Syntheseansätzen erfolgt nach allgemein bekannten Methoden entweder durch Ausfällen aus dem Reaktionsmedium mittels Elektrolyten, wie beispielsweise Natriumchlorid oder Kaliumchlorid, oder durch Eindampfen der Reaktionslösung, beispielsweise durch Sprühtrocknung, wobei dieser Reaktionslösung eine Puffersubstanz zugeführt werden kann. Die Verbindungen (1) haben faserreaktive Eigenschaften und besitzen sehr gute Farbstoffeigenschaften. Sie können deshalb zum Färben von hydroxy- und/oder carbonamidgruppenhaltigem Material, insbesondere Fasermaterial, wie auch von Leder, verwendet werden. Ebenso können auch die bei der Synthese der erfindungsgemäßen Verbindungen anfallenden Lösungen, gegebenenfalls nach Zusatz einer Puffersubstanz und gegebenenfalls nach Konzentrierung, direkt als Flüssigpräparation der färberischen Verwendung zugeführt werden.

Gegenstand der vorliegenden Erfindung ist deshalb auch die Verwendung der Verbindungen (1) zum Färben von hydroxy- und carbonamidgruppenahltigen Materialien bzw. Verfahren zu deren Anwendung auf diesen Substraten. Eingeschlossen sind hierbei die Massefärbung, beispielsweise Folien aus Polyamid, und die Druckfärbung. Bevorzugt kommen die Materialien in Form von Fasermaterialien zur Anwendung, insbesondere als Textilfasermaterialien, wie in Form von Geweben oder als Garne, wie in Form von Strängen und Wickelkörpern.

Hydroxygruppenhaltige Materialien sind natürliche oder synthetische hydroxygruppenahltige Materialien, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und viskose Kunstseide.

Carbonamidgruppenhaltige Materialien sind beispielsweise synthetische und natürliche Polyamide und Polyurethane, insbesondere in Form der Fasern, beispielsweise Wolle und andere Tierhaare, Seide, Leder, Polyamid-6,6, Polyamid-6, Polyamid-11 und Polyamid-4.

Die Verbindungen (1) lassen sich auf den genannten Substraten, insbesondere auf den genannten Fasermaterialien, nach den für wasserlösliche Farbstoffe, insbesondere für faserreaktive Farbstoffe, bekannten Anwendungstechniken applizieren und fixieren. So erhält man mit ihnen auf Cellulosefasern nach den Ausziehverfahren aus langer Flotte unter Verwendung von verschiedensten säurebindenden Mitteln und gegebenenfalls neutralen Salzen, wie Natriumchlorid oder Natriumsulfat, Färbungen mit sehr guten Farbausbeuten sowie ausgezeichnetem Farbaufbau bei hohen Fixiergraden. Man färbt bei Temperaturen zwischen 40 und 105°C, gegebenenfalls bei Temperaturen bis zu 120°C unter Druck, und gegebenenfalls in Gegenwart von üblichen Färbereihilfsmitteln im wäßrigen Bad. Man kann dabei so vorgehen, daß man das Material in das warme Bad einbringt und dieses allmählich auf die gewünschte Färbetemperatur erwärmt und den Färbeprozeß bei dieser Temperatur zu Ende führt. Die das Ausziehen der Verbindungen (1) beschleunigenden Neutralsalze können dem Bade gewünschtenfalls auch erst nach Erreichen der eigentlichen Färbetemperatur zugesetzt werden.

Nach dem Klotzverfahren werden auf Cellulosefasern ebenfalls ausgezeichnete Farbausbeuten mit hohen Fixiergraden und ein sehr guter Farbaufbau erhalten, wobei durch Verweilen bei Raumtemperatur oder erhöhter Temperatur, beispielsweise bis zu etwa 60°C, durch Dämpfen oder mit Trockenhitze in üblicher Weise fixiert werden kann.

Ebenfalls erhält man nach den üblichen Druckverfahren für Cellulosefasern, die entweder einphasig durchgeführt werden können, beispielsweise durch Bedrucken mit einer Natriumcarbonat oder ein anderes säurebindendes Mittel und die Verbindung (1) enthaltenden Druckpaste und durch anschließendes Dämpfen bei 100 bis 103°C, oder die zweiphasig, beispielsweise durch Bedrucken mit neutraler oder schwach saurer, das Farbmittel enthaltenden Druckpaste und anschließendes Fixieren entweder durch Hindurchführen der bedruckten Ware durch ein heißes elektkrolythaltiges alkalisches Bad oder durch Überklotzen mit einer alkalischen elektkrolythaltigen Klotzflotte mit anschließendem Verweilen dieses behandelten Materials oder anschließendem Dämpfen oder anschließender Behandlung mit Trockenhitze, durchgeführt werden können, farbstarke Drucke mit gutem Stand der Konturen und einem klaren Weißfond. Der Ausfall der Drucke ist von wechselnden Fixierbedingungen nur wenig abhängig. Sowohl in der Färberei als auch in der Druckerei sind die mit den Verbindungen (1) erhaltenen Fixiergrade sehr hoch.

Bei der Fixierung mittels Trockenhitze nach den üblichen Thermofixierverfahren verwendet man Heißluft von 120 bis 200°C. Neben dem üblichen Wasserdampf von 101 bis 103°C kann auch überhitzter Dampf und Druckdampf von Temperaturen bis zu 160°C eingesetzt werden.

Die säurebindenden und die Fixierung der Verbindungen (1) auf den Cellulosefasern bewirkenden Mittel sind beispielsweise wasserlösliche basische Salze der Alkalimetalle und der Erdalkalimetalle von anorganischen oder organilschen Säuren, ebenso Verbindungen, die in der Hitze Alkali freisetzen. Insbesondere sind die Alkalilmetallhydroxide und Alkalimetallsalze von schwachen bis mittelstarken anorganischen oder organischen Säuren zu nennen, wobei von den Alkaliverbindungen vorzugsweise die Natrium- und Kaliumverbindungen gemeint sind. Solche säurebindenden Mittel sind beispielsweise Natriumhydroxid, Kaliumhydroxid, Natriumcarbonat, Natriumbicarbonat, Kaliumcarbonat, Natriumformiat, Natriumdihydrogenphosphat, Dinatriumhydrogenphosphat, Natriumtrichloracetat, Wasserglas oder Trinatriumphosphat.

Durch die Behandlung der Verbindungen (1) mit den säurebindenden Mitteln, gegebenenfalls unter Wärmeeinwirkung, werden diese chemisch an die Cellulosefaser gebunden; insbesondere die Cellulosefärbungen zeigen nach der üblichen Nachbehandlung durch Spülen zur Entfernung von nicht fixierten Anteilen der Verbindungen (1) ausgezeichnete Naßechtheiten, zumal sich solche nicht fixierten Anteile leicht wegen ihrer guten Kaltwasserlöslichkeit auswaschen lassen.

Die Färbungen auf Polyurethan- und Polyamidfasern werden üblicherweise aus saurem Milieu ausgeführt. So kann man beispielsweise dem Färbebad Essigsäure und/oder Ammoniumsulfat und/oder Essigsäure und Ammonmiumacetat oder Natriumacetat zufügen, um den gewünschten pH-Wert zu erhalten. Zwecks Erreichung einer brauchbaren Egalität der Färbung empfiehlt sich ein Zusatz an üblichen Egalisierhilfsmitteln, wie beispielsweise auf Basis eines Umsetzungsproduktes von Cyanurchlorid mit der dreifachen molaren Menge einer Aminobenzolsulfonsäure und/oder einer Aminonaphthalinsulfonsäure oder auf Basis eines Umsetzungsproduktes von beispielsweise Stearylamin mit Ethylenoxid. In der Regel wird das zu färbende Material bei einer Temperatur von etwa 40°C in das Bad eingebracht, dort einige Zeit darin bewegt, das Färbebad dann auf den gewünschten schwach sauren, vorzugsweise schwach essigsauren, pH-Wert nachgestellt und die eigentliche Färbung bei einer Temperatur zwischen 60 und 98°C durchgeführt. Die Färbungen können aber auch bei Siedetemperatur oder bei Temperaturen bis zu 120°C (unter Druck) ausgeführt werden.

Die mit den Verbindungen (1) hergestellten Färbungen und Drucke zeichnen sich durch klare Nuancen aus. Insbesondere die Färbungen und Drucke auf Cellulosefasermaterialien besitzen, wie bereits erwähnt, darüber hinaus eine hohe Farbstärke, eine gute Lichtechtheit und sehr gute Naßechtheiten, wie Wasch-, Walk-, Wasser-, Seewasser-, Überfärbe- und Schweißechtheiten, des weiteren eine gute Plissierechtheit, Bügelechtheit und Reibechtheit.

Besonders hervorzuheben sind die mit den erfindungsgemäßen Farbstoffen auf Cellulosefasermaterialien erzielbaren hohen Fixierausbeuten, die bei der Anwendung nach Druckverfahren und Klotzfärbeverfahren über 90 % betragen können. Ein weiterer Vorteil der Verbindungen (1) besteht in der leichten Auswaschbarkeit der beim Druck- oder Färbevorgang nicht fixierten Anteile, wodurch der Waschvorgang der bedruckten oder gefärbten Cellulosefasermaterialien mit geringen Waschflottenmengen und gegebenenfalls einer energiesparenden Temperaturführung während des Waschvorganges bewerkstelligt werden kann.

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säure angegeben; im allgemeinen werden sie in Form ihrer Alkalimetallsalze, wie Lithium-, Natrium oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Farbstoffe angegebenen Absorptionsmaxima (λₘₐₓ) im sichtbaren Bereich wurden anhand ihrer Alkalimetallsalze in wäßriger Lösung ermittelt. In den Tabellenbeispielen sind die λₘₐₓ-Werte bei der Farbtonangabe in Klammern gesetzt; die Wellenlängenangabe bezieht sich auf nm.

### Beispiel 1

Zu einer Lösung mit einem pH-Wert von 6 von 50,1 Teilen der Ausgangs-Kupplungskomponente 1-(2'-Morpholino-4'-fluor-1',3',5'-triazin-6'-yl)-amino-3,6-disulfo-8-hydroxy-naphthalin in 500 Teilen Wasser gibt man langsam unter gutem Rühren innerhalb von etwa 10 Minuten eine auf üblichem Wege hergestellte wäßrige, saure Diazoniumsalzsuspension von 28,1 Teilen 4-(β-Sulfatoethylsulfonyl)-anilin hinzu und führt die Kupplungsreaktion unter Einhaltung eines pH-Wertes von 4 bis 5 bei einer Temperatur von 10 bis 15°C durch. Nach etwa einer Stunde stellt man den pH-Wert des Reaktionsansatzes auf 6 und isoliert die erfindungsgemäße Monoazoverbindung in üblicher Weise, beispielsweise durch Sprühtrocknung oder durch Aussalzen mit Natriumchlorid.

Die erfindungsgemäße Azoverbindung besitzt, in Form der freien Säure geschrieben, die Formel

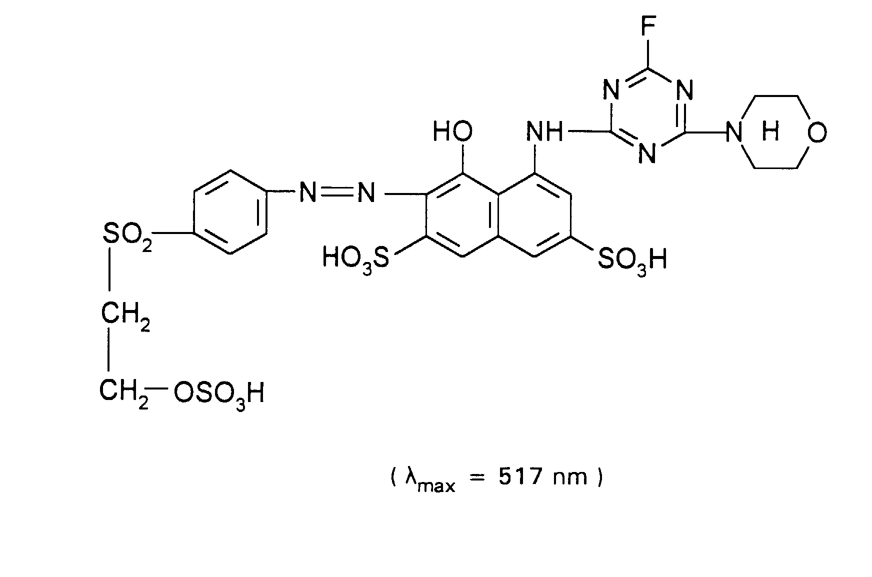

Die erfindungsgemäße Monoazoverbindung besitzt sehr gute faserreaktive Farbstoffeigenschaften und färbt die in der Beschreibung genannten Materialien, wie insbesondere Cellulosefasermaterialien, wie Baumwolle, nach den in der Technik für faserreaktive Farbstoffe üblichen Anwendungs- und Fixierverfahren in klaren roten Tönen mit guten Echtheitseigenschaften, von denen insbesondere die guten Licht- und Wasserechtheiten hervorgehoben werden können.

### Beispiele 2 bis 19

In den nachfolgenden Tabellenbeispielen werden weitere erfindungsgemäße Monoazoverbindungen entsprechend der allgemeinen Formel (A) 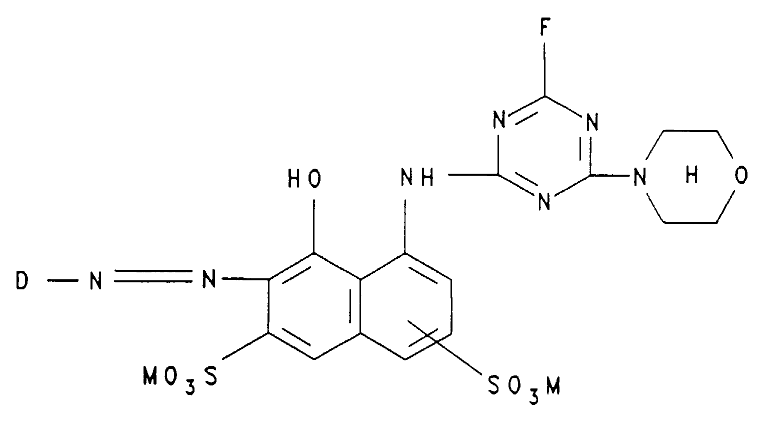 (worin M die für Formel (1) genannte Bedeutung besitzt, anhand ihrer Komponenten beschrieben. Sie lassen sich in erfindungsgemäßer Weise aus den aus der allgemeinen Formel (A) ersichtlichen Ausgangsverbindungen (der Diazokomponente D-NH₂ entsprechend der Anilinverbindung der allgemeinen Formel (3), Cyanurfluorid, Morpholin und 1-Amino-3,6- oder -4,6-disulfo-8-hydroxynapthalin) herstellen. Sie besitzen sehr gute faserreaktive Farbstoffeigenschaften und färben die in der Beschreibung genannten Materialien, wie insbesondere Cellulosefasermaterialien, in dem in dem jeweiligen Tabellenbeispiel (hier für Baumwolle) angegebenen Farbton in hoher Farbstärke und guten Echtheiten.

| | Azoverbindung der Formel (A) | | |
|---|---|---|---|
| Beispiel | Rest D | Stellung der Gruppe -SO₃M | Farbton |
| 2 | 4-Vinylsulfonyl-phenyl | meta | rot (516) |
| 3 | 3-(β-Sulfatoethylsulfonyl)-phenyl | meta | rot (514) |
| 4 | 2-Sulfo-4-(β-sulfatoethylsulfonyl)-phenyl | meta | rot (510) |
| 5 | 2-Methoxy-5-(β-sulfatoethylsulfonyl)-phenyl | meta | rotviolett (540) |
| 6 | 2-Hydroxy-5-(β-sulfatoethylsulfonyl)-phenyl | meta | rotviolett (538) |
| 7 | 2,5-Dimethoxy-4-(β-sulfatoethylsulfonyl)-phenyl | meta | rotviolett (537) |
| 8 | 2-Methoxy-5-methyl-4-(β-sulfatoethylsulfonyl)-phenyl | meta | rot (534) |
| 9 | 2-Chlor-5-(β-sulfatoethylsulfonyl)-phenyl | meta | rot (512) |
| 10 | 2-Carboxy-5-(β-sulfatoethylsulfonyl)-phenyl | meta | rot (513) |
| 11 | 4-Vinylsulfonyl-phenyl | para | rot (513) |
| 12 | 3-(β-Sulfatoethylsulfonyl)-phenyl | para | rot |
| 13 | 2-Sulfo-4-(β-sulfatoethylsulfonyl)-phenyl | para | rot |
| 14 | 2-Methoxy-5-(β-sulfatoethylsulfonyl)-phenyl | para | rot |
| 15 | 2-Hydroxy-5-(β-sulfatoethylsulfonyl)-phenyl | para | rot |
| 16 | 2,5-Dimethoxy-4-(β-sulfatoethylsulfonyl)-phenyl | para | rot |
| 17 | 2-Methoxy-5-methyl-4-(β-sulfatoethylsulfonyl)-phenyl | para | rot |
| 18 | 2-Chlor-5-(β-sulfatoethylsulfonyl)-phenyl | para | rot |
| 19 | 2-Carboxy-5-(β-sulfatoethylsulfonyl)-phenyl | meta | rot (513) |

## Patentansprüche

1. Monoazoverbindung entsprechend der allgemeinen Formel (1) 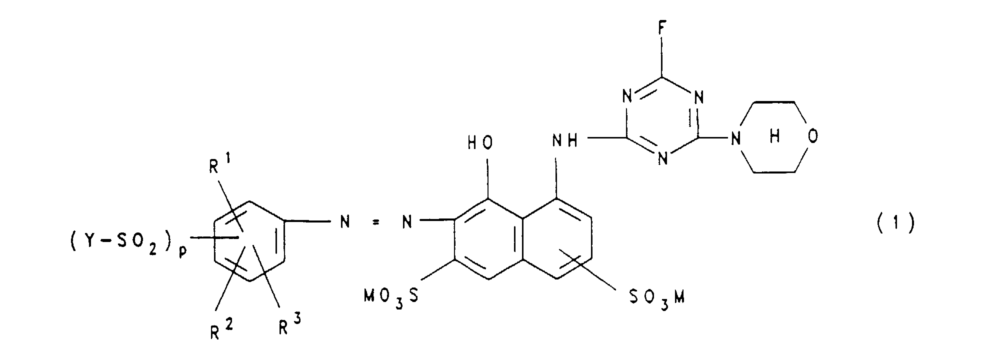 in welcher bedeuten:
M ist Wasserstoff oder ein Alkalimetall;
R¹ ist Wasserstoff oder Sulfo;
R² ist Wasserstoff, Alkyl von 1 bis 4 C-Atomen, Alkoxy von 1 bis 4 C-Atomen, Sulfo, Hydroxy, Halogen oder Carboxy;
R³ ist Wasserstoff, Halogen, Alkyl von 1 bis 4 C-Atomen oder Alkoxy von 1 bis 4 C-Atomen;
Y ist Vinyl oder ist Ethyl, das in β-Stellung einen Substituenten enthält, der durch Alkali unter Bildung der Vinylgruppe eliminierbar ist;
p ist die Zahl 1 oder 2;
die eine Gruppe -SO₃M steht in para- oder meta-Stellung zur Aminogruppe an den Naphthalinring gebunden.

2. Monoazoverbindung nach Anspruch 1 entsprechend der Formel 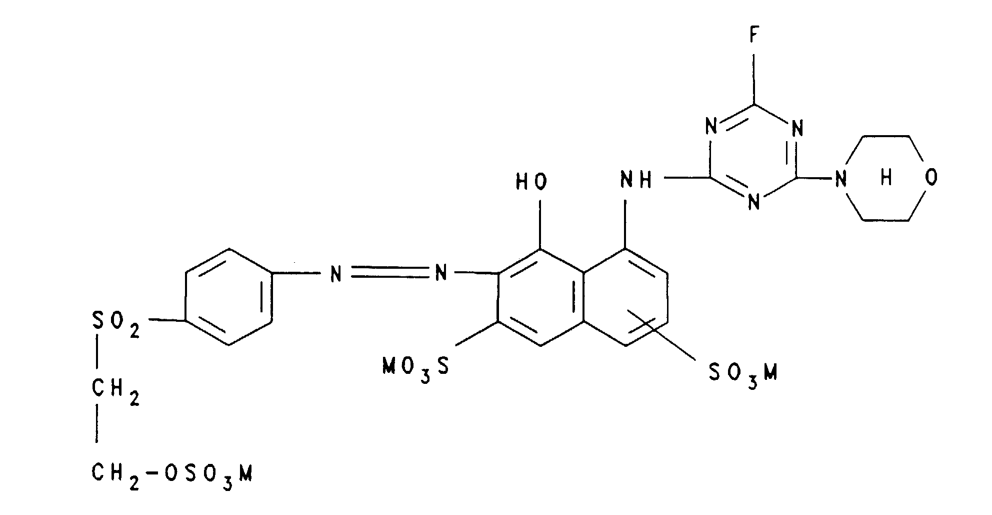 in welcher M die in Anspruch 1 genannte Bedeutung besitzt.

3. Verfahren zur Herstellung einer Monoazoverbindung der allgemeinen Formel (1) von Anspruch 1, dadurch gekennzeichnet, daß man eine Verbindung der allgemeinen Formel (3) 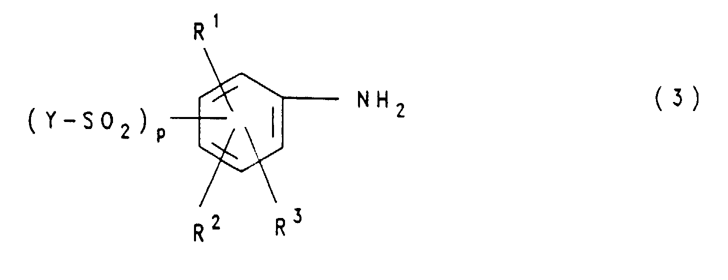 in welcher Y, R¹, R², R³ und p die in Anspruch 1 genannten Bedeutungen haben, diazotiert und mit einer Verbindung der allgemeinen Formel (4) 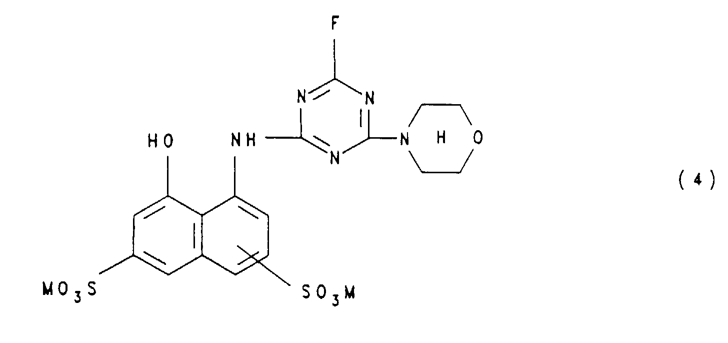 in welcher M die in Anspruch 1 genannte Bedeutung besitzt, kuppelt.

4. Verwendung einer Monoazoverbindung von Anspruch 1 oder einer nach Anspruch 3 hergestellten Monoazoverbindung zum Färben (einschließlich Bedrucken) von hydroxy- und/oder carbonamidgruppenhaltigem Material, insbesondere Fasermaterial.

5. Verfahren zum Färben (einschließlich Bedrucken) von hydroxy- und/oder carbonamidgruppenhaltigem Material, insbesondere Fasermaterial, bei welchem man einen Farbstoff auf das Material aufbringt und den Farbstoff auf dem Material mittels Wärme oder mit Hilfe eines alkalisch wirkenden Mittels oder mittels beider Maßnahmen fixiert, dadurch gekennzeichnet, daß man als Farbstoff eine Monoazoverbindung von Anspruch 1 oder eine nach Anspruch 3 hergestellte Monoazoverbindung einsetzt.

## Claims

1. A monoazo compound conforming to the formula (1) 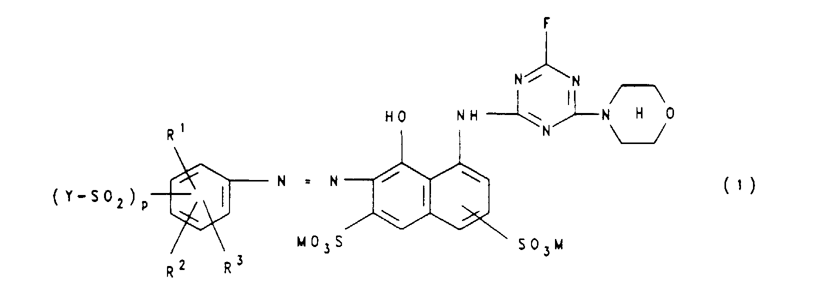 where
M is hydrogen or an alkali metal;
R¹ is hydrogen or sulfo;
R² is hydrogen, alkyl of 1 to 4 carbon atoms, alkoxy of 1 to 4 carbon atoms, sulfo, hydroxyl, halogen or carboxyl;
R³ is hydrogen, halogen, alkyl of 1 to 4 carbon atoms or alkoxy of 1 to 4 carbon atoms;
Y is vinyl or is ethyl which contains in the β-position a substituent which is eliminable with alkali to form a vinyl group;
p is 1 or 2;
the one -SO₃M group is attached to the naphthalene ring para or meta to the amino group.

2. A monoazo compound as claimed in claim 1, conforming to the formula 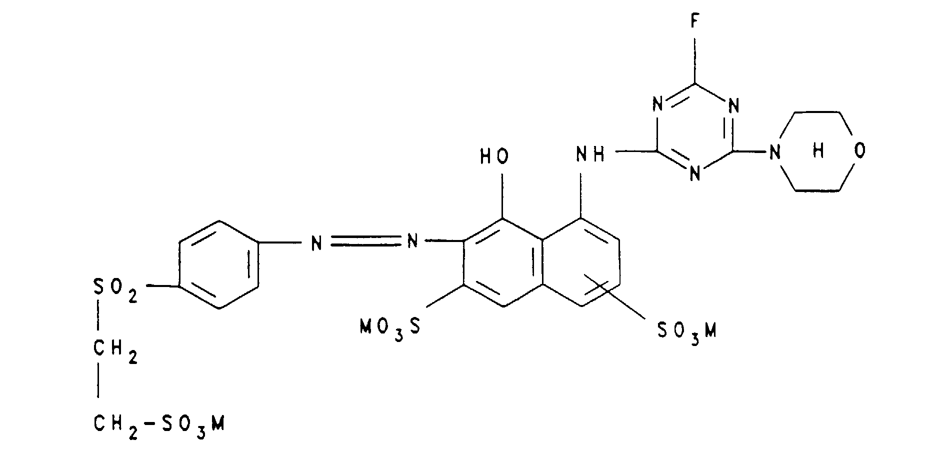 where M is as defined in claim 1.

3. A process for preparing a monoazo compound of the formula (1) of claim 1, which comprises diazotizing a compound of the formula (3) 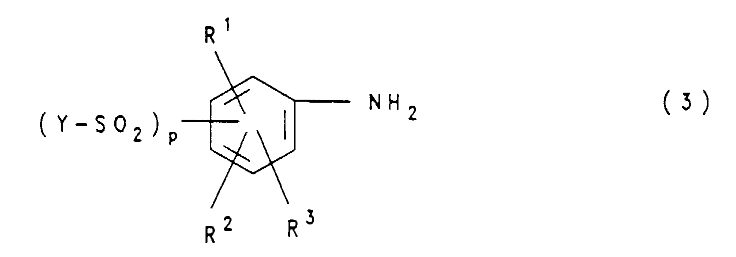 where Y, R¹, R², R³ and p are each as defined in claim 1, and coupling the resulting diazonium compound with a compound of the formula (4) 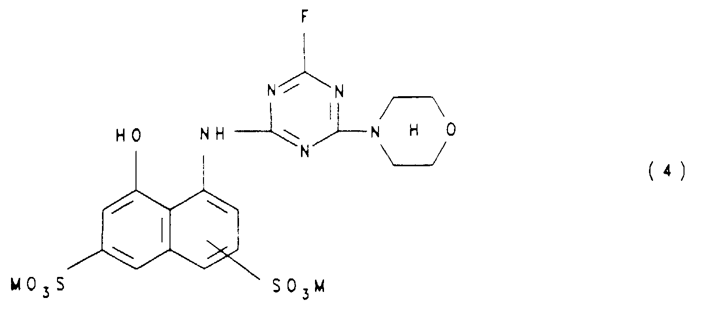 where M is as defined in claim 1.

4. The use of a monoazo compound of claim 1 or of a monoazo compound prepared as claimed in claim 3 for dyeing (including printing) hydroxyl- and/or carboxamido-containing material, in particular fiber material.

5. A process for dyeing (including printing) hydroxyl- and/or carboxamido-containing material, in particular fiber material, by applying a dye to the material and fixing the dye on the material by means of heat or with the aid of an alkaline agent or by means of both measures, which comprises using a dye that is a monoazo compound of claim 1 or a monoazo compound prepared as claimed in claim 3.

## Revendications

1. Composé monoazoïque correspondant à la formule générale (1) : 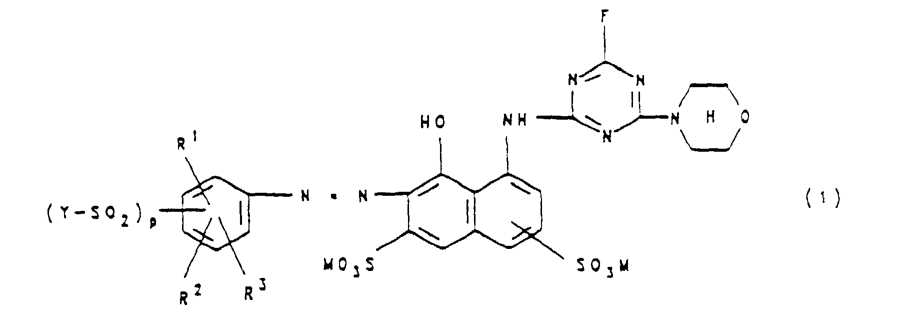 dans laquelle :
M est un hydrogène ou un métal alcalin;
R¹ est un hydrogène ou un sulfo;
R² est un hydrogène, un alkyle de 1 à 4 atomes de carbone, un alcoxy de 1 à 4 atomes de carbone, un sulfo, un hydroxy, un halogène ou un carboxy;
R³ est un hydrogène, un halogène, un alkyle de 1 à 4 atomes de carbone ou un alcoxy de 1 à 4 atomes de carbone;
Y est un vinyle ou est un éthyle, qui porte en position β un substituant qui peut être éliminé en milieu alcalin avec formation du radical vinyle, et
p est le nombre 1 ou 2,
l'un des groupes -SO₃M se situe en position para ou méta par rapport au radical amino fixé sur le cycle naphtalène.

2. Composé monoazoïque suivant la revendication 1, correspondant à la formule : 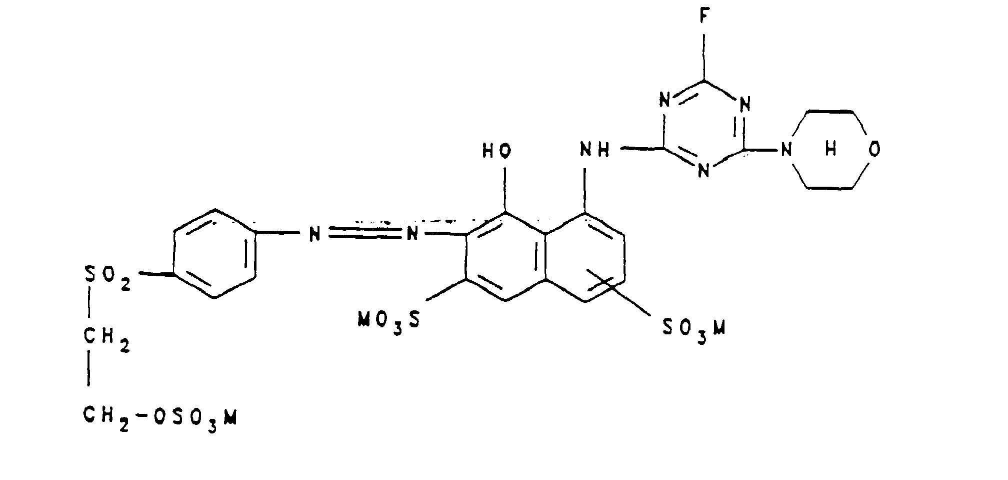 dans laquelle M possède la signification donnée à la revendication 1.

3. Procédé de préparation d'un composé monoazoïque de formule générale (1) de la revendication 1, caractérisé en ce que l'on diazote un composé de formule générale (3) : 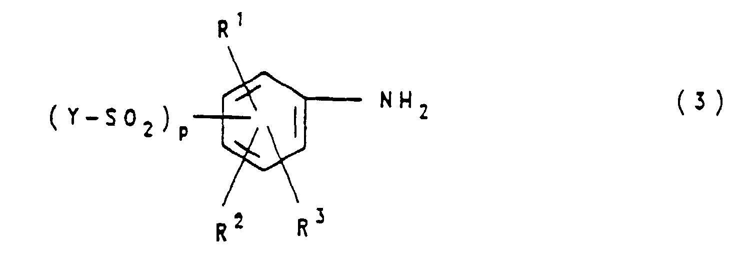 dans laquelle Y, R¹, R², R³ et p ont les significations données à la revendication 1, et en ce qu'on le couple avec un composé de formule générale (4) 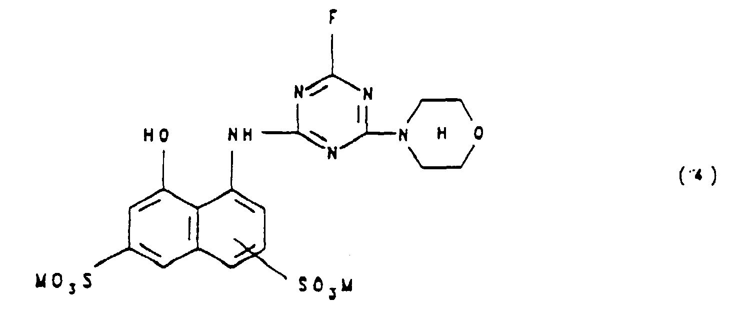 dans laquelle M possède la signification donnée à la revendication 1.

4. Utilisation d'un composé monoazoïque de la revendication 1 ou d'un composé monoazoïque préparé suivant la revendication 3 pour la coloration (y compris l'impression) d'un matériau contenant des radicaux hydroxy et/ou carboxamide, en particulier un matériau fibreux.

5. Procédé de coloration (y compris l'impression) d'un matériau contenant des radicaux hydroxy et/ou carboxamide, en particulier un matériau fibreux, dans lequel on applique un colorant sur le matériau et on fixe le colorant sur le matériau par la chaleur ou à l'aide d'un milieu agissant par alcalinité ou au moyen des deux procédures, caractérisé en ce que le colorant utilisé est un composé monoazoïque de la revendication 1 ou un composé monoazoïque préparé suivant la revendication 3.
